# EUROPEAN PATENT SPECIFICATION

(11) **EP 3 311 835 B1**
(45) Date of publication and mention of the grant of the patent: **24.03.2021**
(21) Application number: 17187097.5
(22) Date of filing: 11.07.2011
(51) Int. Cl.: A61K 31/675, A61K 31/506, A61K 31/404, A61K 31/436, A61K 31/665, A61K 31/44, A61K 39/395, A61P 35/00, A61K 45/06, A61K 31/4045, A61K 31/4412, A61P 35/02, A61P 43/00

(54) **ADMINISTRATION OF HYPOXIA ACTIVATED PRODRUGS AND ANTIANGIOGENIC AGENTS FOR THE TREATMENT OF CANCER**
VERABREICHUNG VON HYPOXIE-AKTIVIERTEN PRODRUGS UND ANTIANGIOGENEN WIRKSTOFFEN ZUR BEHANDLUNG VON KREBS
ADMINISTRATION DE PROMÉDICAMENTS ACTIVÉS PAR HYPOXIE ET D'AGENTS ANTI-ANGIOGÉNIQUES POUR LE TRAITEMENT DU CANCER

(30) Priority: 12.07.2010 US 363610 P; 31.03.2011 US 201161470412 P; 01.04.2011 US 201161470812 P
(43) Date of publication of application: 25.04.2018
(62) Divisional of application: 11807356.8
(73) Proprietor: Threshold Pharmaceuticals Inc., San Francisco, CA 94080 (US)
(72) Inventor: KROLL, Stewart, South San Francisco, CA 94080 (US); CURD, John, South San Francisco, CA 94080 (US); HART, Charles, South San Francisco, CA 94080 (US); SUN, Jessica, South San Francisco, CA 94080 (US)
(74) Representative: Forresters IP LLP

(56) References cited:
- WO-A1-2008/033041
- WO-A1-2010/048330
- WO-A2-2007/002931
- WO-A2-2009/126705
- U. EMMENEGGER: "Low-Dose Metronomic Daily Cyclophosphamide and Weekly Tirapazamine: A Well-Tolerated Combination Regimen with Enhanced Efficacy That Exploits Tumor Hypoxia", CANCER RESEARCH, vol. 66, no. 3, 1 February 2006 (2006-02-01), pages 1664-1674, XP055084237, ISSN: 0008-5472, DOI: 10.1158/0008-5472.CAN-05-2598
- ANONYMOUS: "Guidance for Industry Estimating the Maximum Safe Starting Dose in Initial Clinical Trials for Therapeutics in Adult Healthy Volunteers Department of Health and Human Services Food and Drug Administration Center for Drug Evaluation and Research (CDER)", FDA GUIDELINES, 1 July 2005 (2005-07-01), XP055152598,
- Joachim Drevs ET AL: "Antiangiogenic Potency of Various Chemotherapeutic Drugs for Metronomic Chemotherapy", Anticancer Research, 1 May 2004 (2004-05-01), page 1759, XP055679366, Greece Retrieved from the Internet: URL:http://ar.iiarjournals.org/content/24/ 3A/1759.full.pdf
- MAHARJAN ET AL: "Anti-Angiogenic Effect of Orally Available Pemetrexed for Metronomic Chemotherapy", PHARMACEUTICS, vol. 11, no. 7, 13 July 2019 (2019-07-13), page 332, XP055679484, DOI: 10.3390/pharmaceutics11070332
- MITESH BORAD ET AL: "Complete Phase 1B Study of TH-302 in Combination with Gemcitabine, Docetaxel or Pemetrexed", EUROPEAN SOCIETY FOR MEDICAL ONCOLOGY (ESMO) 35TH ANNUAL MEETING; MILAN, ITALY; 8-12 OCTOBER 2010, 1 October 2010 (2010-10-01), - 12 October 2010 (2010-10-12), pages 1-1, XP055143265, Switzerland

## Description

### FIELD OF THE INVENTION

The present invention relates to hypoxia activated prodrugs and antiangiogenic agents for use in methods of treating cancer and generally relates to the fields of medicine, pharmacology, and medicinal chemistry.

### BACKGROUND OF THE INVENTION

Antiangiogenic agents have been used for treating various cancers. Administration of these agents often shows encouraging progression free survival (PFS). However, the overall survival (OS) periods for cancer patients on such treatment are often comparable to survival periods observed for treatments employing agents other than antiangiogenic agents. For example, in breast cancer, the median PFS for a combination of Avastin® bevacizumab (Roche) and paclitaxel and paclitaxel alone was, in one study, 11.3 and 5.8 months, respectively. However, the OS for the combination of bevacizumab and paclitaxel and paclitaxel alone was 24.8 and 26.5 months, respectively.

In another example, from a metastatic renal cell carcinoma (mRCC) study, the median PFS for a combination of bevacizumab and interferon-a and a combination of placebo and interferon was 10.2 and 5.4 months, respectively. However, the OS for the combination of bevacizumab and the interferon and the combination of placebo and the interferon was 21 and 23 months, respectively. In a third example, from an RCC study, the median PFS for Sutent® sunitinib (Pfizer) and IFN-α and IFN-α alone was 11 and 5 months, respectively. However, the OS for the combination of sunitinib and IFN-α and IFN-α alone was 26.4 and 21.8 months, respectively. In another example, from an RCC treatment, the median PFS for Nexavar® sorafenib (Bayer) and placebo was 24 and 12 weeks, respectively. However, the OS for sorafenib and placebo was 17.8 and 14.3 months, respectively.

Antiangiogenic agents prevent or target vascularization of tumor tissues and so can be predicted to increase tumor hypoxia, which is highly associated with poor prognosis. Tumor hypoxia has been targeted in cancer therapy research for many years, but without success, using hypoxia activated prodrugs. Most recently, a hypoxia activated prodrug called TH-302 (see PCT Pub. Nos. 2007/002931, 2008/083101, and 2010/048330, and PCT App. No. US2011/042047) has showed promising activity in Phase 2 clinical trials, but to date, no hypoxia activated prodrug has been approved by the FDA.

Theoretically, the efficacy of a hypoxia activated prodrug might be improved by co-administration with an antiangiogenic agent, as one could predict that the increase in hypoxia in the tumor microenvironment due to administration of the antiagiogenic agent would increase activation of the hypoxia activated prodrug in the hypoxic tumor zone. Conversely, however, it has been hypothesized that the initial effect of certain antiangiogenic therapy is vascular normalization mediated by the antiangiogenic first acting on immature vasculature, characterized by poor pericyte coverage. This would result in decreased hypoxia and decreased activation of a hypoxia activated prodrug. This initial effect is predicted to be followed by overall vascular inhibition, leading to greater tumor hypoxia.

However, one could as well predict that the decreased vascularization of the tumor as a result of antiangiogenic therapy would decrease delivery of the hypoxia activated prodrug to the hypoxic zone, leading to reduced efficacy of the hypoxia activated prodrug. Moreover, administration of antiangiogenic agents can increase tumor hypoxia and lead to the emergence of aggressive solid tumor phenotypes (for bevacizumab, see Rapisarda et al. Mol. Cancer Ther. 8: 1867-77, 2009; for sorafenib, see Chang et al. Cancer Chemother. Pharmacol. 59: 561-574, 2007, for sunitinib, see Paez-Ribes et al. Cancer Cell 15: 220-231, 2009 and Ebos et al., Cancer Cell 15: 232-239, 2009, for motesanib see Kruser et al. Clin. Cancer Res. 16: 3639-3647, 2010, and for DC101, see Franco et al. Cancer Res. 66: 3639-3648, 2006). These more aggressive phenotypes could be resistant to hypoxia activated prodrug therapy as well.

Thus, there is a need for improved treatments for cancer using antiangiogenic agents. The present invention meets this need by providing combinations for treating cancer in which a hypoxia activated prodrug and an antiangiogenic agent are co-administered.

### SUMMARY

The invention is defined in the claims. Provided herein are combinations for treating cancer comprising administering a therapeutically effective amount of a hypoxia activated prodrug and a therapeutically effective amount of an antiangiogenic agent. In some disclosures, the antiangiogenic agent is administered sufficiently before the administration of the hypoxia activated prodrug that the hypoxic fraction of the tumor is increased at the time of hypoxia activated prodrug administration. In various disclosures, the hypoxia activated prodrug is a compound of formula I: 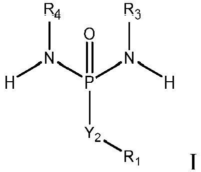 wherein Y₂ is O, S, NR₆, NCOR₆, or NSO₂R₆ wherein R₆ is C₁-C₆ alkyl, C₁-C₆ heteroalkyl, aryl, or heteroaryl; R₃ and R₄ are independently selected from the group consisting of 2-haloalkyl, 2-alkylsulfonyloxyalkyl, 2-heteroalkylsulfonyloxyalkyl, 2-arylsulfonyloxyalkyl, and 2-heteroalkylsulfonyloxyalkyl; R₁ has the formula L-Z₃; L is C(Z₁)₂; each Z₁ independently is hydrogen, halogen, C₁-C₆ alkyl, C₁-C₆ heteroalkyl, aryl, heteroaryl, C₃-C₈ cycloalkyl, heterocyclyl, C₁-C₆ acyl, C₁-C₆ heteroacyl, aroyl, or heteroaroyl; or L is: 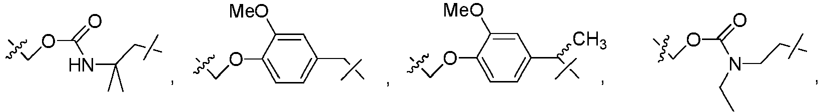 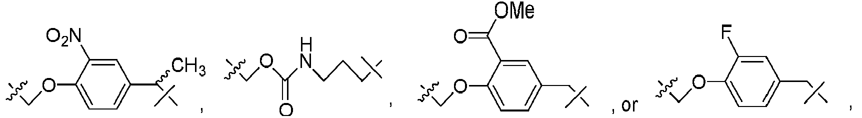 Z₃ is a bioreductive group having a formula selected from the group consisting of: 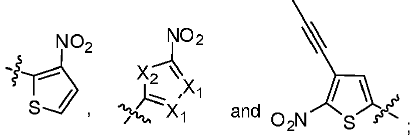 wherein each X₁ is independently N or CR₈; X₂ is NR₇, S, or O; each R₇ is independently C₁-C₆ alkyl, C₁-C₆ heteroalkyl, C₃-C₈ cycloalkyl, heterocyclyl, aryl or heteroaryl; and R₈ is independently hydrogen, halogen, cyano, CHF₂, CF₃, CO₂H, amino, C₁-C₆ alkyl, C₁-C₆ heteroalkyl, C₁-C₆ cycloalkyl, C₁-C₆ alkoxy, C₁-C₆ alkylamino, C₁-C₆ dialkylamino, aryl, CON(R₇)₂, C₁-C₆ acyl, C₁-C₆ heteroacyl, aroyl or heteroaroyl; or a pharmaceutically acceptable salt thereof. In various aspects of the disclosure the compound is a compound of formula I that is TH-281, TH-302, or TH-308 (structures provided below).

In the invention, the antiangiogenic agent is selected from the group consisting of everolimus, pazopanib, sorafenib, and sunitinib. In one aspect of the disclosure bevacizumab and TH-302 or another hypoxia activated prodrug are administered to a patient with a cancer that is resistant to bevacizumab therapy alone (i.e., the cancer has progressed despite having been treated with bevacizumab). In one embodiment, TH-302 and pazopanib are for use in administration in combination to treat a cancer selected from the group consisting of renal cell carcinoma (RCC), sarcoma, and pancreatic cancer, including but not limited to pancreatic neuroendocrine tumors (PNET). In one embodiment, TH-302 and sorafenib are for use in administration in combination to treat a cancer selected from the group consisting of hepatic cell carcinoma (HCC) and RCC. In one embodiment, TH-302 and sunitinib are for use in administration in combination to treat a cancer selected from the group consisting of RCC, including advanced RCC, gastrointestinal cancer, including but not limited to gastrointestinal stromal tumor (GIST), and pancreatic cancer, including PNET.

The disclosed methods are useful for treating various cancers including solid tumors. In various embodiments of the disclosure, a biomarker of hypoxia is used to select patients for treatment and/or to identify patients that are responding to therapy comprising a hypoxia activated prodrug and an antiangiogenic agent. When used to select patients, these methods provide that increased levels of biomarkers that increase with hypoxia (or decreased levels of those that decrease with hypoxia) correlate with increased probability that the patient will respond favorably to therapy. When used to monitor treatment, these methods provide that decreased levels of biomarkers associated with hypoxia correlate with a favorable response to therapy.

These and other aspects and embodiments of the invention are described in additional detail below.

### DETAILED DESCRIPTION

The practice of the present invention includes the use of conventional techniques of molecular biology (including recombinant techniques), microbiology, cell biology, biochemistry and immunology, which are within the skill of the art.

### Definitions

In this specification and in the claims that follow, reference will be made to a number of terms that shall be defined to have the meanings below. All numerical designations, *e.g.*, pH, temperature, time, concentration, and weight, including ranges, are approximations that typically may be varied ( + ) or ( - ) by increments of 0.1, 1.0, or 10.0, as appropriate. All numerical designations may be understood as preceded by the term "about". Reagents described herein are exemplary and equivalents of such may be known in the art.

The singular form "a", "an", and "the" includes plural references unless the context clearly dictates otherwise.

The term "comprising" means any recited elements are necessarily included and other elements may optionally be included. "Consisting essentially of' means any recited elements are necessarily included, elements that would materially affect the basic and novel characteristics of the listed elements are excluded, and other elements may optionally be included. "Consisting of' means that all elements other than those listed are excluded. Embodiments defined by each of these terms are within the scope of this invention.

Certain terms related to formula I are defined below.

"Acyl" refers to -CO- alkyl, wherein alkyl is as defined here.

"Aroyl" refers to -CO-aryl, wherein aryl is as defined here.

"Alkoxy" refers to -O-alkyl, wherein alkyl is as defined here.

"Alkenyl" refers to a linear monovalent hydrocarbon radical or a branched monovalent hydrocarbon radical having the number of carbon atoms indicated in the prefix and containing at least one double bond, but no more than three double bonds. For example, (C₂-C₆)alkenyl includes, ethenyl, propenyl, 1,3-butadienyl and the like. Alkenyl can be optionally substituted with substituents, including for example, deuterium ("D"), hydroxyl, amino, mono or di(C₁-C₆)alkyl amino, halo, C₂-C₆ alkenyl ether, cyano, nitro, ethynyl, C₁-C₆ alkoxy, C₁-C₆ alkylthio, -COOH, -CONH₂, mono- or di(C₁-C₆)alkylcarboxamido, -SO₂NH₂, -OSO₂-(C₁-C₆)alkyl, mono or di(C₁-C₆) alkylsulfonamido, aryl, heteroaryl, alkyl or heteroalkylsulfonyloxy, and aryl or heteroarylsulfonyloxy.

"Alkyl" refers to a linear saturated monovalent hydrocarbon radical or a branched saturated monovalent hydrocarbon radical having the number of carbon atoms indicated in the prefix. (C₁-C₆)alkyl can be optionally substituted with substituents, including for example, deuterium ("D"), hydroxyl, amino, mono or di(C₁-C₆) alkyl amino, halo, C₂-C₆ alkenyl ether, cyano, nitro, ethenyl, ethynyl, C₁-C₆ alkoxy, C₁-C₆ alkylthio, -COOH, -CONH₂ , mono- or di(C₁-C₆)alkylcarboxamido, -SO₂NH₂, -OSO₂-(C₁-C₆)alkyl, mono or di(C₁-C₆) alkylsulfonamido, aryl, heteroaryl, alkylsulfonyloxy, heteroalkylsulfonyloxy, arylsulfonyloxy or heteroarylsulfonyloxy.

As used in this disclosure, the prefixes (C₁-C_{qq}), C_{1-qq}, and C₁-C_{qq}, wherein qq is an integer from 2-20, have the same meaning. For example, (C₁-C₆)alkyl, C₁-₆ alkyl, or C₁-C₆ alkyl includes methyl, ethyl, n-propyl, 2-propyl, n-butyl, 2-butyl, tert-butyl, pentyl, and the like. For each of the definitions herein (e.g., alkyl, alkenyl, alkoxy, etc.), when a prefix is not included to indicate the number of main chain carbon atoms in an alkyl portion, the radical or portion thereof will have six or fewer main chain carbon atoms.

"Alkylamino" or mono-alkylamino refers to -NH-alkyl, wherein alkyl is as defined here.

"Alkynyl" refers to a linear monovalent hydrocarbon radical or a branched monovalent hydrocarbon radical having the number of carbon atoms indicated in the prefix and containing at least one triple bond, but no more than two triple bonds. For example, (C₂-C₆)alkynyl includes, ethynyl, propynyl, and the like. Alkynyl can be optionally substituted with substituents, including for example, deuterium ("D"), hydroxyl, amino, mono or di(C₁-C₆)alkyl amino, halo, C₂-C₆ alkenyl ether, cyano, nitro, ethenyl, C₁-C₆ alkoxy, C₁-C₆ alkylthio, -COOH, -CONH₂, mono- or di(C₁-C₆)alkylcarboxamido, -SO₂NH₂, -OSO₂-(C₁-C₆)alkyl, mono or di(C₁-C₆)alkylsulfonamido, aryl, heteroaryl, alkyl or heteroalkylsulfonyloxy, and aryl or heteroarylsulfonyloxy.

"Aryl" refers to a monovalent monocyclic or bicyclic aromatic hydrocarbon radical of 6 to 10 ring atoms which is substituted independently with one to eight substituents, preferably one, two, three, four of five substituents selected from deuterium ("D"), alkyl, cycloalkyl, cycloalkylalkyl, halo, nitro, cyano, hydroxyl, alkoxy, amino, acylamino, mono-alkylamino, di-alkylamino, haloalkyl, haloalkoxy, heteroalkyl, COR (where R is hydrogen, alkyl, cycloalkyl, cycloalkyl-alkyl, phenyl or phenylalkyl), -(CR'R")ₙ-COOR (where n is an integer from 0 to 5, R' and R" are independently hydrogen or alkyl, and R is hydrogen, alkyl, cycloalkyl, cycloalkylalkyl, phenyl or phenylalkyl) or -(CR'R")ₙ-CONR^{x}R^{y} (where n is an integer from 0 to 5, R' and R" are independently hydrogen or alkyl, and R^{x} and R^{y} are independently selected from hydrogen, alkyl, cycloalkyl, cycloalkylalkyl, phenyl or phenylalkyl). In one disclosure, R^{x} and R^{y} together is cycloalkyl or heterocyclyl. More specifically the term aryl includes, but is not limited to, phenyl, biphenyl, 1-naphthyl, and 2-naphthyl, and the substituted forms thereof.

"Cycloalkyl" refers to a monovalent cyclic hydrocarbon radical of three to seven ring carbons. The cycloalkyl group can have one or more double bonds and can also be optionally substituted independently with one, two, three or four substituents selected from alkyl, optionally substituted phenyl, or -C(O)R^{z} (where R^{z} is hydrogen, alkyl, haloalkyl, amino, mono-alkylamino, di-alkylamino, hydroxyl, alkoxy, or optionally substituted phenyl). More specifically, the term cycloalkyl includes, for example, cyclopropyl, cyclohexyl, cyclohexenyl, phenylcyclohexyl, 4-carboxycyclohexyl, 2-carboxamidocyclohexenyl, 2-dimethylaminocarbonyl-cyclohexyl, and the like.

"Dialkylamino" or di-alkylamino refers to -N(alkyl)₂, wherein alkyl is as defined here.

"Heteroalkyl" refers to an alkyl radical as defined herein with one, two or three substituents independently selected from cyano, -OR^{w}, -NR^{x}R^{y}, and -S(O)ₚR^{z} (where p is an integer from 0 to 2), with the understanding that the point of attachment of the heteroalkyl radical is through a carbon atom of the heteroalkyl radical. R^{w} is hydrogen, alkyl, cycloalkyl, cycloalkyl-alkyl, aryl, aralkyl, alkoxycarbonyl, aryloxycarbonyl, carboxamido, or mono- or di-alkylcarbamoyl. R^{x} is hydrogen, alkyl, cycloalkyl, cycloalkyl-alkyl, aryl or araalkyl. R^{y} is hydrogen, alkyl, cycloalkyl, cycloalkyl-alkyl, aryl, araalkyl, alkoxycarbonyl, aryloxycarbonyl, carboxamido, mono- or di-alkylcarbamoyl or alkylsulfonyl. R^{z} is hydrogen (provided that n is 0), alkyl, cycloalkyl, cycloalkyl-alkyl, aryl, araalkyl, amino, mono-alkylamino, di-alkylamino, or hydroxyalkyl. Representative examples include, for example, 2-hydroxyethyl, 2,3-dihydroxypropyl, 2-methoxyethyl, benzyloxymethyl, 2-cyanoethyl, and 2-methylsulfonyl-ethyl. For each of the above, R^{w}, R^{x}, R^{y}, and R^{z} can be further substituted by amino, halo, fluoro, alkylamino, di-alkylamino, OH or alkoxy. Additionally, the prefix indicating the number of carbon atoms (e.g., C₁-C₁₀) refers to the total number of carbon atoms in the portion of the heteroalkyl group exclusive of the cyano, -OR^{w}, -NR^{x}R^{y}, or-S(O)ₚR^{z} portions. In one disclosure, R^{x} and R^{y} together is cycloalkyl or heterocyclyl.

"Heteroaryl" refers to a monovalent monocyclic, bicyclic or tricyclic radical of 5 to 12 ring atoms having at least one aromatic ring containing one, two, or three ring heteroatoms selected from N, O, or S, the remaining ring atoms being C, with the understanding that the attachment point of the heteroaryl radical will be on an aromatic ring. The heteroaryl ring is optionally substituted independently with one to eight substituents, preferably one, two, three or four substituents, selected from alkyl, cycloalkyl, cycloalkyl-alkyl, halo, nitro, cyano, hydroxyl, alkoxy, amino, acylamino, mono-alkylamino, di-alkylamino, haloalkyl, haloalkoxy, heteroalkyl, -COR (where R is hydrogen, alkyl, phenyl or phenylalkyl, - (CR'R")ₙ-COOR (where n is an integer from 0 to 5, R' and R" are independently hydrogen or alkyl, and R is hydrogen, alkyl, cycloalkyl, cycloalkyl-alkyl, phenyl or phenylalkyl), or-(CR'R")ₙ-CONR^{x}R^{y} (where n is an integer from 0 to 5, R' and R" are independently hydrogen or alkyl, and R^{x} and R^{y} are, independently of each other, hydrogen, alkyl, cycloalkyl, cycloalkyl-alkyl, phenyl or phenylalkyl). In one disclosure, R^{x} and R^{y} together is cycloalkyl or heterocyclyl. More specifically the term heteroaryl includes, but is not limited to, pyridyl, furanyl, thienyl, thiazolyl, isothiazolyl, triazolyl, imidazolyl, isoxazolyl, pyrrolyl, pyrazolyl, pyridazinyl, pyrimidinyl, benzofuranyl, tetrahydrobenzofuranyl, isobenzofuranyl, benzothiazolyl, benzoisothiazolyl, benzotriazolyl, indolyl, isoindolyl, benzoxazolyl, quinolyl, tetrahydroquinolinyl, isoquinolyl, benzimidazolyl, benzisoxazolyl or benzothienyl, indazolyl, pyrrolopyrymidinyl, indolizinyl, pyrazolopyridinyl, triazolopyridinyl, pyrazolopyrimidinyl, triazolopyrimidinyl, pyrrolotriazinyl, pyrazolotriazinyl, triazolotriazinyl, pyrazolotetrazinyl, hexaaza-indenly, and heptaaza-indenyl and the derivatives thereof. Unless indicated otherwise, the arrangement of the hetero atoms within the ring can be any arrangement allowed by the bonding characteristics of the constituent ring atoms.

"Heterocyclyl" or "cycloheteroalkyl" refers to a saturated or unsaturated nonaromatic cyclic radical of 3 to 8 ring atoms in which one to four ring atoms are heteroatoms selected from O, NR (where R is hydrogen, alkyl, cycloalkyl, cycloalkylalkyl, phenyl or phenylalkyl), P(=O)OR^{w}, or S(O)ₚ (where p is an integer from 0 to 2), the remaining ring atoms being C, wherein one or two C atoms can optionally be replaced by a carbonyl group. The heterocyclyl ring can be optionally substituted independently with one, two, three or four substituents selected from alkyl, aryl, arylalkyl, heteroaryl, heteroarylalkyl, cycloalkyl, cycloalkylalkyl, halo, nitro, cyano, hydroxyl, alkoxy, amino, mono-alkylamino, dialkylamino, haloalkyl, haloalkoxy, -COR (where R is hydrogen, alkyl, cycloalkyl, cycloalkylalkyl, phenyl or phenylalkyl), -(CR'R")ₙ-COOR (n is an integer from 0 to 5, R' and R" are independently hydrogen or alkyl, and R is hydrogen, alkyl, cycloalkyl, cycloalkylalkyl, phenyl or phenylalkyl), or -(CR'R")ₙ-CONR^{x}R^{y} (where n is an integer from 0 to 5, R' and R" are independently hydrogen or alkyl, R^{x} and R^{y} are, independently of each other, hydrogen, alkyl, cycloalkyl, cycloalkylalkyl, phenyl or phenylalkyl). More specifically the term heterocyclyl includes, but is not limited to, pyridyl, tetrahydropyranyl, N-methylpiperidin-3-yl, N-methylpyrrolidin-3-yl, 2-pyrrolidon-1-yl, furyl, quinolyl, thienyl, benzothienyl, pyrrolidinyl, piperidinyl, morpholinyl, pyrrolidinyl, tetrahydrofuranyl, tetrahydrothiofuranyl, 1,1-dioxo-hexahydro-1Δ⁶-thiopyran-4-yl, tetrahydroimidazo[4,5-c]pyridinyl, imidazolinyl, piperazinyl, and piperidin-2-only and the derivatives thereof. The prefix indicating the number of carbon atoms (e.g., C₃-C₁₀) refers to the total number of carbon atoms in the portion of the cycloheteroalkyl or heterocyclyl group exclusive of the number of heteroatoms.

"Heteroacyl" refers to -CO-heteroalkyl, wherein heteroalkyl is as defined here.

"Heteroaroyl" refers to -CO-heteroayl, wherein heteroaryl is as defined here.

"Rₛᵤₗ sulfonyloxy" refers to Rₛᵤₗ-S(=O)₂-O- and includes alkylsulfonyloxy, heteroakylsulfonyloxy, cycloalkylsulfonyloxy, heterocyclylsulfonyloxy, arylsulfonyloxy and heteroarylsulfonyloxy wherein Rₛᵤₗ is alkyl, heteroakyl, cycloalkyl, heterocyclyl, aryl and heteroaryl respectively, and wherein alkyl, heteroakyl, cycloalkyl, heterocyclyl, aryl and heteroaryl are as defined here. Examples of alkylsulfonyloxy include Me-S(=O)₂-O-, Et-S(=O)₂-O-, CF₃-S(=O)₂-O- and the like, and examples of arylsulfonyloxy include: 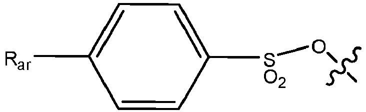 wherein Rₐᵣ is H, methyl, or bromo.

"Substituents" refers to, along with substituents particularly described in the definition of each of the groups above, those selected from: deuterieum, -halogen, -OR',-NR'R", -SR', -SiR'R"R''',-OC(O)R', -C(O)R', -CO₂R', -CONR'R", -OC(O)NR'R",-NR"C(O)R', -NR'-C(O)NR"R''', -NR"C(O)₂R', -NH-C(NH₂)=NH, -NR'C(NH)=NH, -NH-C(NH₂)=NR', -S(O)R', -S(O)₂R', -S(O)₂NR'R", -NR'S(O)₂R", -CN, -NO₂, -R', -N₃, perfluoro(C₁-C₄)alkoxy, and perfluoro(C₁-C₄)alkyl, in a number ranging from zero to the total number of open valences on the radical; and where R', R" and R'" are independently selected from hydrogen, C₁₋₈ alkyl, C₃₋₆ cycloalkyl, C₂₋₈ alkenyl, C₂₋₈ alkynyl, unsubstituted aryl and heteroaryl, (unsubstituted aryl)-C₁-₄ alkyl, and unsubstituted aryloxy-C₁-₄ alkyl, aryl substituted with 1-3 halogens, unsubstituted C₁-₈ alkyl, C₁-₈alkoxy or C₁-₈ thioalkoxy groups, or unsubstituted aryl-C₁-₄ alkyl groups. When R' and R" are attached to the same nitrogen atom, they can be combined with the nitrogen atom to form a 3-, 4-, 5-, 6-, or 7-membered ring. For example, -NR'R" is meant to include 1-pyrrolidinyl and 4-morpholinyl. Other suitable substituents include each of the above aryl substituents attached to a ring atom by an alkylene tether of from 1-4 carbon atoms. Two of the substituents on adjacent atoms of the aryl or heteroaryl ring may optionally be replaced with a substituent of the formula -T²-C(O)-(CH₂)_{q}-U³-, wherein T² and U³ are independently -NH-, -O-, -CH₂- or a single bond, and q is an integer of from 0 to 2. Alternatively, two of the substituents on adjacent atoms of the aryl or heteroaryl ring may optionally be replaced with a substituent of the formula -A-(CH₂)ᵣ-B-, wherein A and B are independently -CH₂-, -O-, -NH-, -S-, -S(O)-, -S(O)₂-,-S(O)₂NR'- or a single bond, and r is an integer of from 1 to 3. One of the single bonds of the new ring so formed may optionally be replaced with a double bond. Alternatively, two of the substituents on adjacent atoms of the aryl or heteroaryl ring may optionally be replaced with a substituent of the formula -(CH₂)ₛ-X⁵-(CH₂)ₜ-, wherein s and t are independently integers of from 0 to 3, and X⁵ is -O-, -NR'-, -S-, -S(O)-, -S(O)₂-, or -S(O)₂NR'-. The substituent R' in-NR'- and -S(O)₂NR'- is selected from hydrogen or unsubstituted C₁-₆ alkyl.

Certain compounds utilized in the present disclosure possess asymmetric carbon atoms (optical centers) or double bonds; the racemates, diastereomers, geometric isomers, regioisomers and individual isomers (e.g., separate enantiomers) are all intended to be encompassed within the scope of the present disclosure. The compounds of the present disclosure may also contain unnatural proportions of atomic isotopes at one or more of the atoms that constitute such compounds. For example, the compounds may be radiolabeled with radioactive isotopes, such as for example, and without limitation, tritium (³H), iodine-125 (¹²⁵I), or carbon-14 (¹⁴C). All isotopic variations of the compounds of the present disclosure, whether radioactive or not, are intended to be encompassed within the scope of the present disclosure.

Other terms related to this invention are defined below.

"Administering" or "administration of' a drug to a patient (and grammatical equivalents of this phrase) refers to direct administration, which may be administration to a patient by a medical professional or may be self-administration, and/or indirect administration, which may be the act of prescribing a drug. For example, a physician who instructs a patient to self-administer a drug and/or provides a patient with a prescription for a drug is administering the drug to the patient.

"Angiogenesis" refers to the growth of new blood vessels from existing blood vessels.

"Antiangiogenic agent" refers to a drug or an agent that can inhibit angiogenesis and includes, without limitation, an anti-VEGF antibody, a VEGF-trap, an anti-VEGFR antibody, a VEGFR inhibitor, or a biological equivalent of each thereof; thalidomide or a thalidomide derivative; a D114-Notch inhibitor; an anti-tubulin vascular disrupting agent (VDA); an angiopoietin-Tie2 inhibitor; a nitric oxide synthase (NOS) inhibitor; a cationic poly amino acid dendrimer; rapamycin (sirolimus) or a rapamycin derivative (including but not limited to everolimus and temsirolimus); a low molecular weight heparin; a SPARC (osteonectin) peptide; bevacizumab, Lucentis® ranibizumab (Roche), ramucirumab (Lilly), Zaltrap® aflibercept, VEGF-trap (Regeneron), interleukin 17 (IL-17), or a biological equivalent of each thereof; DC101; sunitinib; sorafenib; Votrient® pazopanib (GSK); Motesanib® AMG706 (Amgen); Recentin® cediranib (AstraZeneca); Caprelsa® vandetanib (AstraZeneca); Vargatef® BIBF 1120 (Boehringer- Ingelheim); Brivanib® BMS-582664 (BMS); Carbozantinib® XL-184 (Exelixis); Axitinib AG-013736 (Pfizer); Tivozanib AV-951 (Aveo, Astellas); Revlimid® lenalidomide (Celgene); 5,6-dimethylxanthenone-4-acetic acid (DMXAA); nadroparin, 2,5-dimethyl-celecoxib; cyclophosphamide; the Ca++/calmodulin antagonist 4-{3,5-bis-[2-(4-hydroxy-3-methoxy-phenyl)-ethyl]-4,5-dihydro-pyrazol-1-yl}-benzoic acid (HBC); and tasquinimod (quinoline-3-carboxamide). In addition, conventional chemotherapeutic agents such as docetaxel, irinotecan, topotecan, and temozolomide dosed in a manner termed "metronomic dosing" (more frequent than standard administration of lower doses than standard dose levels) are antiangiogenic agents (see Wu et al. Cancer Chemother Pharmacol. 2011 Feb 3. [Epub ahead of print]; Takano et al. J Neurooncol. 2010 Sep;99: 177-185, 2010; Merritt et al. Cancer Biol Ther. 8: 1596-1603, 2009; Sarmiento et al. Onkologie 31: 161-162, 2008; Kim et al. Oncol Rep. 16: 33-39, 2006; and Gille et al. J Dtsch Dermatol Ges. 3: 26-32, 2005).

"Biological equivalent," in reference to an antibody or a fragment thereof, refers to proteins or peptides that bind to the same epitope, as the reference antibody or fragment.

"Cancer" refers to malignant solid tumors of potentially unlimited growth, as well as various blood cancers that may originate from cancer stem cells in the hypoxic bone marrow, which can expand locally by invasion and systemically by metastasis. Examples of cancers include, but are not limited to cancer of the adrenal gland, bone, brain, breast, bronchi, colon and/or rectum, gallbladder, gastrointestinal tract, head and neck, kidneys, larynx, liver, lung, neural tissue, pancreas, prostate, parathyroid, skin, stomach, and thyroid. Other examples of cancers include, adenocarcinoma, adenoma, basal cell carcinoma, cervical dysplasia and in situ carcinoma, Ewing's sarcoma, epidermoid carcinomas, giant cell tumor, glioblastoma multiforma, hairy-cell tumor, intestinal ganglioneuroma, hyperplastic corneal nerve tumor, islet cell carcinoma, Kaposi's sarcoma, leiomyoma, leukemias, lymphomas, malignant carcinoid, malignant melanomas, malignant hypercalcemia, marfanoid habitus tumor, medullary carcinoma, metastatic skin carcinoma, mucosal neuroma, myelodisplastic syndrome, myeloma, mycosis fungoides, neuroblastoma, osteosarcoma, osteogenic and other sarcoma, ovarian tumor, pheochromocytoma, polycythermia vera, primary brain tumor, small-cell lung tumor, squamous cell carcinoma of both ulcerating and papillary type, seminoma, soft tissue sarcoma, retinoblastoma, rhabdomyosarcoma, renal cell tumor or renal cell carcinoma, veticulum cell sarcoma, and Wilm's tumor. Examples of cancers also include astrocytoma, a gastrointestinal stromal tumor (GIST), a glioma or glioblastoma, renal cell carcinoma (RCC), hepatocellular carcinoma (HCC), and a pancreatic neuroendocrine cancer.

"Combination therapy" or "combination treatment" refers to the use of two or more drugs in therapy, i.e., use of a hypoxia activated prodrug as described herein together with one or more antiangiogenic drugs used to treat cancer is a combination therapy. Administration in "combination" refers to the administration of two agents (e.g., a hypoxia activated prodrug and an antiangiogenic agent for treating cancer) in any manner in which the pharmacological effects of both manifest in the patient at the same time. Thus, administration in combination does not require that a single pharmaceutical composition, the same dosage form, or even the same route of administration be used for administration of both agents or that the two agents be administered at precisely the same time. For example, and without limitation, it is contemplated that an antiangiogenic agent can be administered with a hypoxia activated prodrug in accordance with the present invention in combination therapy.

"Hyperproliferative disease" refers to a disease characterized by cellular hyperproliferation (e.g., an abnormally increased rate or amount of cellular proliferation). A cancer is a hyperproliferative disease. Examples of hyperproliferative diseases other than solid tumors include, but are not limited to, allergic angiitis and granulomatosis (Churg-Strauss disease), asbestosis, asthma, atrophic gastritis, benign prostatic hyperplasia, bullous pemphigoid, coeliac disease, chronic bronchitis and chronic obstructive airway disease, chronic sinusitis, Crohn's disease, demyelinating neuropathies, dermatomyositis, eczema including atopic dermatitis, eustachean tube diseases, giant cell arteritis, graft rejection, hypersensitivity pneumonitis, hypersensitivity vasculitis (Henoch-Schonlein purpura), irritant dermatitis, inflammatory hemolytic anemia, inflammatory neutropenia, inflammatory bowel disease, Kawasaki's disease, multiple sclerosis, myocarditis, myositis, nasal polyps, nasolacrimal duct diseases, neoplastic vasculitis, pancreatitis, pemphigus vulgaris, primary glomerulonephritis, psoriasis, periodontal disease, polycystic kidney disease, polyarteritis nodosa, polyangitis overlap syndrome, primary sclerosing cholangitis, rheumatoid arthritis, serum sickness, surgical adhesions, stenosis or restenosis, scleritis, scleroderma, strictures of bile ducts, strictures (of duodenum, small bowel, and colon), silicosis and other forms of pneumoconiosis, type I diabetes, ulcerative colitis, ulcerative proctitis, vasculitis associated with connective tissue disorders, vasculitis associated with congenital deficiencies of the complement system, vasculitis of the central nervous system, and Wegener's granulomatosis.

"Hypoxia activated prodrug" refers to a drug that is less active or inactive under normoxia than under hypoxia or anoxia. Hypoxia activated prodrugs include drugs that are activated by a variety of reducing agents and reducing enzymes, including without limitation single electron transferring enzymes (such as cytochrome P450 reductases) and two electron transferring (or hydride transferring) enzymes (see US Pat. App. Pub. Nos. 2005/0256191, 2007/0032455, and 2009/0136521, and PCT Pub. Nos. 2000/064864, 2004/087075, and 2007/002931). The hypoxia activated prodrugs disclosed herein are compounds of formula I, including but not limited to compounds where Z₃, as defined by that formula, is a 2-nitroimidazole moiety. Examples of particular hypoxia activated prodrugs useful in the methods of the disclosure include without limitation TH-281, TH-302, and TH-308. Methods of synthesizing, formulating, and using TH-302 and other compounds of formula I are described in PCT Pub. Nos. 2007/002931, 2008/083101, and 2010/048330, and PCT App. No. US2011/042047.

"Hypoxic fraction" refers to the ratio in a tumor, a tumor segment, or another cancer, of cells that contain a partial pressure of oxygen (pO₂) of less than or equal to 10 mm Hg over the total number of cells in the tumor, tumor segment, or cancer. Hypoxic fraction can be expressed in percentage by multiplying hypoxic fraction with 100. "Increased hypoxic fraction" refers to, e.g., at least 2%, at least 3%, at least 4%, at least 5%, at least 8%, at least 10%, at least 15%, at least 20%, or at least 25% increase in hypoxic fraction. Alternatively, hypoxic fraction and increased hypoxic fraction can be determined by correlation to a level of a hypoxic marker. In this embodiment, the number or percentage of hypoxic cells in a tumor, tumor segment, or another cancer is not determined; rather, the level of such a hypoxic marker is used to assign a hypoxic fraction corresponding thereto.

"Patient" or "subject" refers to mammals, particularly humans, but also includes animals of veterinary and research interest, such as simians, cattle, horses, dogs, cats, and rodents suffering from cancer or another hyperproliferative disease.

"Pharmaceutically acceptable salt" refers to pharmaceutically acceptable salts derived from a variety of organic and inorganic counter ions well known in the art that include, by way of example only, sodium, potassium, calcium, magnesium, ammonium, and tetraalkylammonium, and when the molecule contains a basic functionality, salts of organic or inorganic acids, such as hydrochloride, hydrobromide, tartrate, mesylate, acetate, maleate, and oxalate. Suitable salts include those described in Stahl and Wermuth (Eds.), Handbook of Pharmaceutical Salts Properties, Selection, and Use; 2002.

QnD or qnd refers to drug administration once every n days. For example, QD (or qd) refers to once every day or once daily dosing, Q2D (or q2d) refers to a dosing once every two days, Q7D refers to a dosing once every 7 days or once a week, Q5D refers to dosing once every 5 days.

"Reduction" of a symptom or symptoms (and grammatical equivalents of this phrase) refers to decreasing the severity or frequency of the symptom(s), or elimination of the symptom(s).

"Relapsed or refractory" refers to a type of cancer that is resistant to treatment with an agent, such as an antiangiogenic agent, or responds to treatment with an agent but recurs with or without being resistant to that agent.

TH-281 refers to the compound of formula: 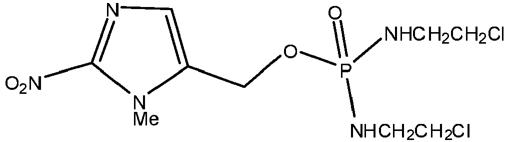 and includes a pharmaceutically acceptable salt thereof.

TH-302 refers to the compound of formula: 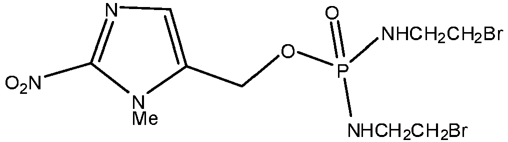 and includes a pharmaceutically acceptable salt thereof.

TH-308 refers to the compound of formula:

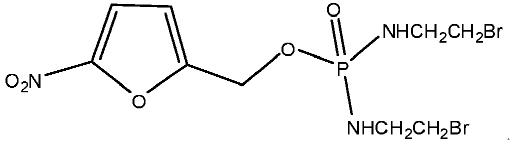

"Therapeutically effective amount" of a drug or an agent refers to an amount of the drug or the agent that, when administered to a patient with cancer or another hyperproliferative disease, will have the intended therapeutic effect, e.g., alleviation, amelioration, palliation or elimination of one or more manifestations of cancer or another hyperproliferative disease in the patient. A therapeutic effect does not necessarily occur by administration of one dose, and may occur only after administration of a series of doses. Thus, a therapeutically effective amount may be administered in one or more administrations.

"Treating" or "treatment of' a condition or patient refers to taking steps to obtain beneficial or desired results, including clinical results. For purposes of this invention, beneficial or desired clinical results include, alleviation or amelioration of one or more symptoms of cancer including conditional survival and reduction of tumor load or volume; diminishment of extent of disease; delay or slowing of disease progression; amelioration, palliation, or stabilization of the disease state; or other beneficial results.

### Treatment Methods

In one aspect, the present disclosure provides a method of treating cancer comprising administering a therapeutically effective amount of a hypoxia activated prodrug and a therapeutically effective amount of an antiangiogenic agent to a patient in need of such treatment thereby treating the cancer. In one disclosure, the combination therapy is administered to a patient that has been previously treated with an antiangiogenic agent, but the cancer is progressing despite the therapy, or the therapy has been discontinued due to cancer progression. In some disclosures, the administration of antiangiogenic agent used in combination therapy is started from at least 24 hours to at least 7 days before administration of the hypoxia activated prodrug. In some disclosures, even longer periods intervene between initiation of antiangiogenic agent and first administration of the hypoxia activated prodrug, including two weeks, one month, six weeks, or longer. After the first administration of the hypoxia activated prodrug, the antiangiogenic agent and the hypoxia activated prodrug may be administered with shorter or even no intervening period of time, i.e., they may both be administered on the same day.

In one disclosure, the hypoxia activated prodrug is selected from the group consisting of TH-281, TH-302, and TH-308. In the present invention, the hypoxia activated prodrug administered is TH-302. In various aspects, the TH-302 or other hypoxia activated prodrug is administered once daily, once every 3 days, weekly, or once every 3 weeks. In one embodiment, the TH-302 or other hypoxia activated prodrug is administered parenterally.

In one aspect of the disclosure the hypoxia activated prodrug is TH-302, which is administered in a daily dose of about 120 mg/m² to about 460 mg/m². In some aspects, the daily dose of TH-302 is administered in a single dose for 5 consecutive days followed by 2 days of no TH-302 administration, i.e., a one week cycle of therapy. Such a 1 week cycle of therapy can be repeated for 1-3 additional cycles, followed by 1-3 weeks of no drug administration, and this treatment regimen may be repeated one or multiple times. The less frequent the administration, the higher the daily doses of the hypoxia activated prodrug administered may be. In one embodiment, TH-302 or other hypoxia activated prodrug is administered once weekly. In one embodiment, the therapeutically effective amount of TH-302 is a once weekly dose of about 480 mg/m² - about 670 mg/m², i.e., 575 mg/m². In another aspect of the disclosure, the therapeutically effective amount of TH-302 is a once weekly dose of about 240 mg/m² administered for three weeks of a 4 week cycle. In one aspect, the therapeutically effective amount of TH-302 is a daily dose of about 240 mg/m² to about 480 mg/m² administered on days 1 and 8 of a 3 week cycle.

In various aspects of the methods of the disclosure, the antiangiogenic agent is selected from the group consisting of bevacizumab (Avastin); pazopanib (Votrient); sorafenib (Nexavar); and sunitinib (Sutent). In various embodiments, the cancer is a cancer of the brain, gastrointestinal tract, kidney, liver, or pancreas. In various embodiments, the cancer is an astrocytoma, a gastrointestinal stromal tumor (GIST), a glioma or glioblastoma, a renal cell carcinoma (RCC), a hepatocellular carcinoma (HCC), or a pancreatic neuroendocrine cancer.

In accordance with some aspects of the methods of the disclosure, when a patient is first administered a hypoxia activated prodrug in combination with an antiangiogenic agent, the antiangiogenic agent is administered first, and the hypoxia activated prodrug is not administered until the antiangiogenic agent has had time to exert its anti-vascular effect and increased hypoxia in (increased the hypoxic fraction of) the tumor. In some disclosures, the antiangiogenic agent will be administered a week before the hypoxia activated prodrug, such as TH-302, is administered. In other disclosures, the antiangiogenic agent is administered at least a day before, 3 days before, 5 days before, or 42 days before a hypoxia activated prodrug such as TH-302 is administered. Typically, there will be additional administrations of the hypoxia activated prodrug and the antiangiogenic agent, and for these subsequent administrations of the two drugs (each "a cycle" of therapy), the delay between administration of the antiangiogenic agent and the next administration of the hypoxia activated prodrug, is not critical, because the antiangiogenic agent has already increased hypoxia in the tumor. In one disclosure, TH-302 or another hypoxia activated prodrug is administered at least 2 hours before the other agent in these second and subsequent cycles of therapy in accordance with the methods of the disclosure. See PCT Pub. No. 2010/048330,. In other aspects, of the methods of the disclosure, the first (and/or one or more subsequent) administration(s) of the hypoxia activated prodrug occurs on the same day as an administration of the antiangiogenic agent.

In one disclosure, the patient's cancer treated is a metastatic cancer or a refractory and/or relapsed cancer that is refractory to first, second, or third line treatment. In another disclosure, the treatment is a first, a second, or a third line treatment. As used herein, the phrase "first line" or "second line" or "third line" refers to the order of treatment received by a patient. First line treatment regimens are treatments given first, whereas second or third line treatment are given after the first line therapy or after the second line treatment, respectively. Therefore, first line treatment is the first treatment for a disease or condition. In patients with cancer, primary treatment can be surgery, chemotherapy, radiation therapy, or a combination of these therapies. First line treatment is also referred to those skilled in the art as primary therapy or primary treatment. Typically, a patient is given a subsequent chemotherapy regimen because the patient did not show a positive clinical or only showed a sub-clinical response to the first line therapy, or the first line treatment has stopped.

It will be appreciated, in view of this disclosure, that the improved treatment results achieved by practice of certain methods of the invention derive from the cancer having an increased hypoxic fraction at the time of first administration of the hypoxia activated prodrug, resulting from the initial administration of the antiangiogenic agent, as compared to the hypoxic fraction of the same cancer in the case where the hypoxia-activated prodrug is administered first or administered prior to the time the antiangiogenic's anti-vascular effects have manifested in the tumor.

In another aspect, the treatment methods of the present disclosure are used for treating hyperproliferative diseases other than cancer.

Methods of preparation of and pharmaceutical compositions of hypoxia activated prodrugs, and other methods of treating cancer by administering various hypoxia activated prodrugs of formula I are described in Duan et al., J. Med. Chem. 2008, 51, 2412-2420, and PCT Pub. Nos. 2007/002931, 2008/083101, and 2010/048330. Other methods of treating cancers, which may be used in combination with the compounds of the present invention, are known to one of skilled in the art, and are described, for example, in the product descriptions found in the 2010 or more current edition of the Physician's Desk Reference, Medical Economics Company, Inc., Oradell, NJ; Goodman and Gilman's The pharmacological basis of therapeutics., Eds. Hardman et al., McGraw-Hill. New York. (US) 2011, 12th Ed., and in publications of the U.S. Food and Drug Administration and the NCCN Guidelines (National Comprehensive Cancer Network). Such methods can be appropriately modified by one of skill in the art, in view of this disclosure, to practice the treatment of the present invention.

In one embodiment, the TH-302 is provided in 100 mg vials, lyophilized, and dissolved in D5W and administered intravenously (IV) over approximately 30 - 60 minutes via an infusion pump. The infusion volume depends on the total dose given (in mg) during the infusion If less than about 1000 mg is being infused, about 500 cc of D5W are used for infusion. If the total dose is greater than about 1000, about 1000 cc of D5W are used for infusion.

The compounds of the invention are now described in the context of combination therapies including particular antiangiogenic agents.

### Combination Treatment with Bevacizumab

In one aspect of the disclosure, the hypoxia activated prodrug is TH-302, which is administered intravenously over about 30 - about 60 minutes in doses of about 240 mg/m² to about 480 mg/m² given in Q2 week (once every 2 weeks) schedule post surgery, for a brain cancer, and the antiangiogenic agent is bevacizumab, which is administered in the standard dose of 10 mg/kg IV once every 2 weeks. This aspect is particularly useful in the treatment of glioblastoma, including recurrent glioblastoma, including recurrent glioblastoma that has been previously treated with bevacizumab or another antiangiogenic agent. Other cancers contemplated to be treated include metastatic colorectal cancer, where the standard bevacizumab doses are 5 mg/kg or 10 mg/kg every 2 weeks in combination with intravenous 5-FU based treatment; lung cancer, including but not limited to non-squamous non-small cell lung cancer; where the standard dose is 15 mg/kg once every 3 weeks in combination with carboplatin and paclitaxel; breast cancer, including metastatic breast cancer, where the standard dose is 10 mg/kg once every 2 weeks in combination with paclitaxel; and renal cell carcinoma, including but not limited to metastatic renal cell carcinoma. In each of these instances, the cancer can include a cancer that has recurred or progressed after prior therapy with bevacizumab or another antiangiogenic agent. Optionally, hypoxia status can be checked using about 500 mg/m² pimonidazole hydrochloride (hypoxyprobe-1) administered IV.

### Combination Treatment with Pazopanib

In one embodiment, the hypoxia activated prodrug TH-302, is administered intravenously over about 30 minutes in doses of about 240 mg/m², about 340 mg/m², about 480 mg/m², or about 575 mg/m² given once weekly or as a 1 week off followed by 3 weeks on therapy schedule or 3 weeks on followed by 1 week off, and the antiangiogenic agent is pazopanib, which is given at the full monotherapy dose of 800 mg (orally) p.o. once daily. Thus, in one embodiment of the invention, the pazopanib is first administered, and after a week of therapy, the hypoxia activated prodrug, TH-302, is first administered a week later in either a weekly or three of four week schedule. In another embodiment of the invention, the pazopanib and TH-302 are first administered together, with TH-302 therapy being administered in either a weekly or three of four week schedule. Other suitable doses of pazopanib on this schedule include 400 mg once daily and 200 mg once daily. This treatment regimen can be administered in accordance with the invention to patients with renal cell carcinoma (RCC), sarcoma, pancreatic cancer, including but not limited to pancreatic neuroendocrine tumors, or other solid tumor cancers.

### Combination Treatment with Sorafenib

In one embodiment, the hypoxia activated prodrug TH-302, is administered on days 8, 15 and 22, of 28 day cycles, and the antiangiogenic agent is sorafenib, which is administered orally without food on days 1-28 of those cycles. In this embodiment, one can treat advanced solid tumor cancers, including but not limited to hepatic cell carcinoma (HCC) and RCC. Sorafenib is dosed at, for example, 200 mg p.o. twice daily (bid, 400 mg total daily dose in two daily doses) on days 1-28, and TH-302 can be dosed at about 240 mg/m² IV (e.g. in a 30 - 60 minute infusion) on days 8, 15 and 22, in cycles of 28 days. Other suitable doses of sorafenib on this schedule include 400 mg p.o. bid, 400 mg p.o. once every day (QD), and 400 mg p.o. once every two days.The sorafenib dose may be reduced to 400 mg once daily or once every other day to manage treatment-related toxicity. Other suitable doses of TH-302 on this schedule include about 180, about 340 and about 480 mg/m² IV.

### Combination Treatment with Sunitinib

In one embodiment, the hypoxia activated prodrug TH-302, is administered on days 8, 15 and 22, of 42 day cycles, and the antiangiogenic agent is sunitinib, which is administered 50 mg PO, daily, on days 1 to 28 and 2 weeks off in the same 42 day cycle. TH-302 can be dosed at about 240 mg/m² IV (e.g., in a 30 - 60 minute infusion) on days 8, 15 and 22, cycles every 42 days. Other suitable doses of TH-302 on this schedule include about 120, about 180, about 340, and about 480 mg/m² IV. In various embodiments, this combination therapy of the invention is administered to a patient selected from the group consisting of RCC, including advanced RCC, gastrointestinal cancer, including but not limited to gastrointestinal stromal tumor (GIST), and pancreatic cancer, including pancreatic neuroendocrine tumor.

In one embodiment, the antiangiogenic agent is a pharmaceutically acceptable salt of sunitinib. In one embodiment, the sunitinib or the pharmaceutically acceptable salt thereof is administered once daily (QD). In one embodiment, the administration of the sunitinib or the pharmaceutically acceptable salt thereof is started at least about 7 days before administering TH-302. In one embodiment, the sunitinib or the pharmaceutically acceptable salt thereof is administered for about 1 week to about 6 weeks before administering the hypoxia activated prodrug. In one embodiment, the sunitinib or the pharmaceutically acceptable salt thereof is administered for about 3 weeks to about 1 year. In one embodiment, the therapeutically effective amount of sunitinib or the pharmaceutically acceptable salt thereof is a daily dose of about 50 mg. In one embodiment, the therapeutically effective amount of sunitinib or the pharmaceutically acceptable salt thereof is in an amount selected from the group of about 25 mg, about 37.5 mg, about 62.5 mg, or about 75 mg. In one embodiment, the sunitinib or the pharmaceutically acceptable salt thereof is administered orally.

### Hypoxic Markers

In various embodiments of the disclosure, a marker of hypoxia (also referred to herein as a "biomarker") is used to select patients for treatment and/or to identify patients that are responding (or not responding) to therapy. Hypoxia markers have been developed in the course of studies showing that hypoxia promotes more aggressive solid tumor phenotypes and associates with resistance to radiation and many chemotherapies, as well as likelihood of tumor invasion and poor patient survival. In particular, cells at pO₂ <10 mm Hg resist the ionizing effect of radiotherapy and cytotoxic effect of chemotherapy. Hypoxic necrotic foci with pseudopalisading tumor cells are one of the features that define glioblastoma (GBM), for example. Thus, a variety of methods have been devised to assess degree of hypoxia in xenografts and patient tumors, and, in accordance with the invention, these methods, suitably modified and practiced as described herein, are used in certain embodiments of the methods of the disclosure to select patients and assess response to therapy. In general, the disclosure provides methods for identifying patients suitable for therapy with a hypoxia activated prodrug in which a marker of hypoxia is used to identify whether a patient's cancer is hypoxic and, if so, then the patient is treated with a hypoxia activated prodrug, i.e., the higher the degree of hypoxia, the more likely the patient will respond to therapy with a hypoxia activated prodrug. Those of skill in the art will appreciate, in view of this disclosure, that these methods are useful in all cancers.

Traditionally, the gold standard for measuring hypoxia has been the use of a polarographic oxygen-sensitive probe, which provides direct measurement of tissue oxygen tension. However, this method has limitations, such as its inability to differentiate between viable and necrotic foci, the inaccessibility of many tumor tissues, including those associated with hematologic malignancies of the bone marrow, and the lack of a practical means to apply the technique in large scale. Pimonidazole and EF5, both 2-nitroimidazole compounds, are hypoxia markers that, via immunohistochemical identification of pimonidazole or EF5 protein adducts, can give a reliable estimate of radio-biologically relevant hypoxia. Molecular oxygen competes with reducing equivalents in a manner such that pimonidazole (and EF5) binding is effectively inhibited at oxygen concentrations above 14 micromolar. This method reliably identifies viable hypoxic cells specifically (necrotic cells cannot metabolize pimonidazole or EF5).

Other hypoxic markers that have been identified in pre-clinical studies that are suitable for use in accordance with the methods of the invention include GLUT-1, HIF-1a, CA-IX, LDH-A, osteopontin, VEGF, and microRNA markers, including but not limited to miR-210. Each of these proteins or RNAs is up-regulated in hypoxia, and they can be detected by tumor biopsy. More conveniently, however, some of these markers, i.e., CA-IX LDH-A, osteopontin, VEGF, and microRNA markers, including but not limited to miR-210 will be detectable in the blood, serum, or plasma of a patient, allowing a simple blood test, instead of a tumor biopsy, to be used to select patients for hypoxia activated prodrug therapy.

In addition, studies have examined the spatial relationship between tumor hypoxia assessed by immunohistochemistry and [18F]-FDG and [18F]-FMISO autoradiography and PET imaging, and these compounds and similar PET tracers, such as [18F]-EF5, [18F]-FAZA, and [18F]-HX4, can be employed in accordance with the methods of the invention. PET tracers such as Copper(II)-diacetyl-bis(*N*⁴-methylthiosemicarbazone) (Cu-ATSM) labeled with a positron emitting isotope of copper [60Cu], [61Cu], [62Cu] or [64Cu], and [68Ga]-1,4,7-Triazacyclononane-1,4,7-triacetic acid-2-nitroimidazole-N-ethylamine (68Ga-NOTA-NI) and [68Ga]-isothiocyanatobenzyl-1,4,7-triazacyclononane-1,4,7-triacetic acid-2-nitroimidazole-N-ethylamine (68Ga-SCN-NOTA-NI) can also be employed in accordance with the methods of the invention. In addition to autoradiography and PET imaging, MRI or EPRI can also be used to detect hypoxia (i.e., to measure the hypoxic fraction of a tumor or otherwise to provide a measure of hypoxia in the cancer). In particular, dynamic contrast-enhanced MRI (DCE-MRI), blood oxygen level-dependent MRI (BOLD-MRI), or diffusion-weighted (DW MRI) can be used to identify hypoxic cancers and thus identify patients ideal for treatment with hypoxia-activated prodrugs.

Hypoxyprobe®-1 (pimonidazole hydrochloride, marketed by Hypoxyprobe, Inc.) when administered, either IV or orally, is distributed to all tissues in the body including the brain but only forms adducts with proteins in those cells that have an oxygen concentration less than 14 micromolar (equivalent to a pO₂ of 10 mm Hg at 37 degrees Celsius). Hypoxyprobe-1MAb1 is a mouse IgG1 monoclonal antibody that detects protein adducts of Hypoxyprobe-1 in hypoxic cells. This reagent is typically added to each tissue sample. Chromogenic or fluorescent secondary antibody reagents are then used in accordance with the invention to reveal where Hypoxyprobe-1 adducts have formed in the hypoxic tissue.

Those of skill in the art will appreciate, in contemplation of this disclosure, that these methods can also be used in accordance with the disclosure to determine if an antiangiogenic agent has had sufficient time to act in a patient treated therewith to increase the hypoxic fraction of a tumor and so to identify when the patient is ready to be first treated with a hypoxia activated prodrug. In this disclosure, an increase in hypoxic fraction, as identified by one of the foregoing methods, signals that administration of the hypoxia activated prodrug may be initiated.

In addition, these methods can be used to identify patients that are responding to therapy with a hypoxia activated prodrug, because the hypoxic fraction of the tumors in such patients should decrease over time, as the hypoxia activated prodrug kills the cells in the hypoxic fraction.

In addition to these markers of hypoxia, there are other markers that can be used to select patients for hypoxia activated prodrug therapy. The hypoxia activated prodrugs of the invention defined by formula I are activated by reductases, so biopsies or blood tests that show a patient has higher levels of an activating reductase, such as POR (P450 oxido-reductase), MTRR (methionine synthase reductase), and/or NOS (nitric oxide synthase), demonstrate that a patient is more likely to respond to hypoxia activated prodrug therapy. Furthermore, the DNA damage induced by these hypoxia activated prodrugs is repaired by the HDR (also known as HR) system, and the lower the levels of the proteins in this system, including but not limited to BRCA, FANC, XPF (also known as ERCC4), XRCC2 and/or XRCC3, in the blood or tumor biopsy of a patient, the more likely the patient will respond to hypoxia activated prodrug therapy with hypoxia activated prodrugs that inflict DNA damage repaired by this system.

Thus, in another aspect, the present disclosure provides a method of determining a cancer patient as likely or unlikely to be suitable for a treatment comprising administration of a hypoxia activated prodrug, the method comprising:
determining the hypoxic fraction in a cancer sample isolated from the cancer patient, optionally including a cancer patient treated with an antiangiogenic agent,
comparing the hypoxic fraction with a predetermined level of hypoxic fraction,
wherein an increased hypoxic fraction compared to the predetermined level indicates the cancer patient as likely to be suitable for the treatment comprising administering the hypoxia activated prodrug, optionally in combination with an antiangiogenic agent, and
a similar or diminished hypoxic fraction compared to the predetermined level indicates the cancer patient as unlikely to be suitable for treatment comprising administering the hypoxia activated prodrug.

As used herein, "a cancer patient as likely or unlikely to be suitable for a treatment" refers to a cancer patient who has undergone, is undergoing, or could undergo the treatment. As used herein, "suitable for a treatment" means that the patient is likely to exhibit one or more desirable clinical outcomes as compared to patients having the same disease and receiving the same treatment but possessing a different characteristic that is under consideration for the purpose of the comparison. In one disclosure, a more desirable clinical outcome is a relatively higher likelihood of favorable tumor response, such as tumor load reduction. In another disclosure, a more desirable clinical outcome is relatively longer overall survival. In another disclosure, a more desirable clinical outcome is relatively longer progression free survival or time to tumor progression. In another disclosure, a more desirable clinical outcome is relatively longer disease free survival. In another embodiment, a more desirable clinical outcome is elimination of, or delay in, tumor recurrence. In another disclosure, a more desirable clinical outcome is elimination of or decreased metastasis. In another disclosuret, a more desirable clinical outcome is lower relative risk. In another embodiment, a more desirable clinical outcome is relatively reduced toxicity or side effects. In some disclosures, more than one clinical outcome is achieved. In one disclosure, a patient possessing a characteristic may exhibit one or more desirable clinical outcomes but simultaneously exhibit one or more less desirable clinical outcomes. The clinical outcomes are then considered collectively, and a decision as to whether the patient is suitable for the therapy will be made accordingly, taking into account the patient's specific situation and the relevance of the respective clinical outcomes. In some disclosures, progression free survival or overall survival is weighted more heavily than tumor response in such decision making.

In one disclosure, the predetermined hypoxic fraction is the hypoxic fraction of a cancer sample that is taken from a cancer patient prior to treatment with any anti-cancer agent, including but not limited to an antiangiogenic agent.

The present disclosure provides a method of determining the cancer patient as unlikely to be suitable for treatment with a hypoxia activated prodrug; in this embodiment, the method reveals that the cancer in the patient is characterized by a small or non-existent hypoxic fraction.

The present disclosure provides a method of determining the cancer patient as likely to be suitable for treatment with a hypoxia activated prodrug. In one disclosure, the method further comprises administering a hypoxia activated prodrug after determining that the patient is likely to respond favorably to treatment. In one disclosure, the hypoxia activated prodrug is TH-281, TH-302, or TH-308. In the invention, the hypoxia activated prodrug is TH-302. In various aspects of the disclosure, the TH-302 is administered once daily, once every 3 days, once weekly, or once every 3 weeks, optionally followed by a week of no therapy. In another embodiment, the TH-302 is administered parenterally. In one aspect of the disclosure, the use further comprises administering to the cancer patient TH-302 or another hypoxia activated prodrug in a daily dose of about 120 mg/m² to about 460 mg/m². Other doses, dosing frequencies, and periods of administration of TH-302 or another hypoxia activated prodrug, as disclosed herein, are also useful in various aspects of the disclosure.

In various disclosures, the antiangiogenic agent is bevacizumab, sunitinib, pazopanib, or sorafenib. Treatment methods carried out as disclosed above are employed for treating the cancer patient as identified above.

In various disclosures, the cancer is a metastatic cancer or a cancer refractory to first, second, or third line treatment, one of which may optionally be a treatment with an antiangiogenic agent.

In one disclosure, the hypoxia activated prodrug selected from the group consisting of TH-281, TH-302, and TH-308. In one disclosure, the predetermined value of the hypoxic fraction is the hypoxic fraction of the same or similar cancer sample from the same or a different patient (including but not limited to predetermined values determined by studies of multiple patients or patient populations). In one disclosure, the predetermined value is determined using a cancer sample from a patient or patients that have not been treated with the antiangiogenic agent.

The hypoxic fraction may be determined by using a variety of known probes and methods, as discussed above. Such methods can include a step of isolating a cancer sample and staining it with a chemical probe of hypoxia such as hypoxyprobe-1 (pimonidazole), EF5, or another 2-nitroimidazole derivative, followed by immunohistochemistry or can alternatively include a step of taking a blood, serum, or plasma sample from a patient and measuring the level of a hypoxic marker contained therein. For example, the level of HIF-1a or carbonic anhydrase can be measured to determine a hypoxic fraction value. Methods for identifying these genes products are known to one of skill in the art. Other suitable hypoxic markers are disclosed above. Other methods include using oxygen electrodes, with or without isolating the cancer sample, to measure oxygen levels. As used herein, "isolating" refers to obtaining biological or cellular materials from a patient.

The present invention having been described in summary and in detail, is illustrated by the following examples.

### EXAMPLES

### Example 1.A. Sunitinib Induced Hypoxia in 786-0 Xenograft Tumor bearing Nude Mice.

To measure the extent of tumor hypoxia and to illustrate methods for increasing the hypoxic fraction of a tumor with an antiangiogenic agent, so as to render the tumor more susceptible to treatment with a hypoxia activated prodrug, nude mice bearing 786-O RCC xenograft tumors were randomized into 3 groups, vehicle (8 mice), sunitinib (Sutent, Pfizer) 20 mg/kg and 40 mg/kg, QDx5, oral, respectively (6 mice each). Seventy one hours after the last sunitinib administration, pimonidazole was administered. Hoechst 33342 was administered 1 hour after administering pimonidazole. About a minute after administering the Hoechst 33342, the animals were sacrificed and tissues harvested. Based on microscopic evaluation of the harvested tissues, a dose-dependent increase in hypoxia and decrease in perfusion were observed. By morphometric analysis, the hypoxic fraction in the 786-O xenograft tumor was 1.6 ± 0.9% with 20 mg/kg and 17.2 ± 0.9% with 40 mg/kg, compared with 1.4 ± 0.9% with vehicle. Significant increased hypoxic fraction was observed when 40 mg/kg of sunitinib was administered. The 786-O RCC tumor is a well-vascularized tumor as characterized by CD31 and Hoechst staining with a relatively small baseline hypoxic compartment (<5% volume) in the xenograft tumors. Still, sunitinib induced a dose dependent increase in tumor hypoxia volume and a corresponding decrease in functional vasculature in the RCC tumor.

### Example 1.B. Sunitinib Induced Hypoxia in H460 Xenograft Tumor Bearing Nude Mice

To measure the extent of tumor hypoxia and to illustrate methods for increasing the hypoxic fraction of a tumor with an antiangiogenic agent, so as to render the tumor more susceptible to treatment with a hypoxia activated prodrug, nude mice bearing H460 non small cell lung cancer (NSCLC) xenograft tumors were intraperitoneally administered sunitinib with the dose of 20 mg/kg and 40 mg/kg, QDx5, respectively (5 mice each), and compared with vehicle treated animals (5 mice). Pimonidazole and Hoechst 33342 were subsequently administered, and the animals sacrificed as described above, and the results again demonstrated a dose-dependent increase in hypoxia and decrease in perfusion. The percent hypoxic fraction in the harvested tissue was analyzed by morphometrics. The NSCLC (H460) tumor exhibits a baseline hypoxic fraction of 7%. Sunitinib induced a dose-dependent increase in tumor hypoxia volume (24 ± 3.2% with 40 mg/kg vs. 7.3 ± 3.8%, with Vehicle, p<0.05) and a corresponding decrease in tumor microvasculature.

### Example 1.C. Sorafenib Induced Hypoxia in 786-0 RCC Xenograft Tumor Bearing Nude Mice

Similar demonstrations were performed in the 786-O RCC using another antiangiogenic agent, sorafenib (Nexavar® sorafenib). Sorafenib 20 mpk (mgs per kilogram) and 40 mpk were orally administered once daily for 7 days. Pimonidazole was administered 3 h after the last sorafenib administration. Hoechst 33342 was administered 1 h after administering pimonidazole. About a minute after administering the Hoechst 33342, the animals were sacrificed and tissues harvested. The 786-O (RCC) tumor exhibits a baseline hypoxic fraction of 0.8%. Sorafenib induced a dose-dependent increase in tumor hypoxia volume: 0.8 ± 0.2% with vehicle; 3.5 ± 1.6% with 20 mg/kg sorafenib administration, and 10 ± 2.5% with sorafenib 40 mg/kg. Significant increased hypoxia volume was observed in the 40 mg/kg treated group (p<0.05 vs. vehicle).

### Example 1.D. Sorafenib Induced Hypoxia in PLC/PRF/5 Xenograft Tumor Bearing SCID Mice

The hypoxia level in another cancer, hepatocellular cancer PLC/PRF/5, after sorafenib administration was also measured. The measurement was carried out as above, except that sorafenib was administered at 20 or 40 mg/kg once daily for 8 days. The PLC/PRF/5 (HCC) tumor exhibits a baseline hypoxic fraction of 4.3%. Sorafenib induced a dose-dependent increase in tumor hypoxia volume: 4.3 ± 0.4% with vehicle; 6.5 ± 0.6% with 20 mg/kg sorafenib administration; and 9.3 ± 0.7% with sorafenib 40 mg/kg. Significant increased hypoxia volume was observed in 20 mg/kg treated group (p<0.05 vs. vehicle) and 40 mg/kg treated group (p<0.001 vs. vehicle).

### Example 2. TH-302 Enhances Antitumor Activity of Antiangiogenic Agents

TH-302's anti cancer efficacy was demonstrated in combination with antiangiogenic therapy. Xenograft tumors were established by *s.c.* implantation of 5 x10⁶ 786-O human renal cell carcinoma (RCC), 5x10⁶ A375 melanoma, or 1x10⁶ H460 human non-small cell lung cancer (NSCLC) cells into the flanks of nude mice, or 5x10⁶ PLC/PRF/5 hepatocellular carcinoma (HCC) into the flanks of Severe Combined Immunodeficient (SCID) mice. Tumor hypoxia was detected by pimonidazole immunostaining, and morphometric analysis was performed to determine the hypoxic fraction. When tumor size was approximately 100-150 mm³, sunitinib or sorafenib was administered daily. Sunitinib at 20, 40, or 80 mg/kg was administered p.o. daily for 3 weeks (QDx21). For all of these studies except the study utilizing the A375 melanoma model, TH-302 administration began one week after antiangiogenic agent administration. In the A375 melanoma model, TH-302 administration was begun on the same day as sorafenib administration. TH-302 at 50 mg/kg was administered i.p. daily for 5 days on and 2 days off (QDx5) for 2 weeks. Sunitinib was administered 4 hours before TH-302 on days when both agents were given.

In the RCC tumor, sunitinib activity was enhanced with TH-302 combination therapy: tumor growth inhibition (TGI) increased from 38% with 40 mg/kg sunitinib monotherapy to 75% with co-administration of 50 mg/kg TH-302.

In the NSCLC tumor, sunitinib (80 mg/kg QDx21) activity was potentiated with TH-302. TGI increased from 78% with 80 mg/kg sunitinib monotherapy to 97% with 50 mg/kg TH-302 combination therapy.

In another study, sunitinib was administered for 5 weeks, and TH-302 was administered for 4 weeks, beginning one week after sunitinib therapy was initiated, in accordance with the methods of the invention. With this longer dosing regimen, anti tumor activity increased. Medium dose sunitinib (40 mg/kg) in combination with TH-302 reached 99% TGI and delayed tumor growth to 500 mm³ for 29 days. Importantly, body weight loss, a toxicity indicator, was not significantly increased with TH-302 in combination with any of the antiangiogenic agents in these studies.

A complementary effect of TH-302 in combination with sorafenib (once daily for 16 days or QDx16) was also observed in multiple xenograft tumors, including PLC/PRF/5 HCC, H460 NSCLC, and A375 melanoma xenografts. For example, 94% TGI for combination therapy was observed, versus less than 80% TGI for either monotherapy in the NSCLC tumor. Sorafenib combination therapy also increased TGI when administered (QDx14) to 786-O RCC bearing mice in combination with TH-302 (QDx5/week for two weeks, 50 mg/kg).

In another study, the effect of TH-302 (dosed at 50 mgs per kg either for 19 days, QDx19, or dosed for 12 days at 50 mpk beginning at Day 8) in combination with everolimus (dosed once daily for 19 days or QDx19) was also observed in 786-O RCC xenografts. For example, 84% TGI for combination therapy was observed, versus less than 50% TGI for everolimus monotherapy or 16% TGI in TH-302 alone group. Interestingly, about the same TGI was observed for both TH-302 treatment arms, demonstrating that delaying the first administration of the TH-302 until after the everolimus had time to increase the hypoxic fraction of the tumor provided enhanced efficacy (i.e., a decrease of almost one third of the total amount of TH-302 administered resulted in similar efficacy as the full dose). The following tables summarize the data obtained in the studies described above.

**Table 1: TH-302 enhanced Sunitinib monotherapy in 786-O renal cell carcinoma**

| | **TGI (%)** | **Max. BW loss (%)** |
|---|---|---|
| **Vehicle** | | **o** |
| **TH-302 50mpk** | **9.9** | **0.1** |
| **Sunitinib 20mpk** | **16.2** | **0** |
| **Sunitinib 40mpk** | **37.9** | **0** |
| **Sunitinib 20mpk + TH-302** | **20.3** | **3.7** |
| **Sunitinib 40mpk + TH-302** | **75*** | **2.4** |

| | | |
|---|---|---|
| *, p<0.05 vs. Vehicle | | |

**Table 2: TH-302 enhanced sunitinib monotherapy in H460 non small cell lung cancer**

| | **Sunitinib 3wks + TH-302 2wks** | | **Sunitinib 5wks + TH-302 4wks** | |
|---|---|---|---|---|
| | **TGI (%)** | **Max. BW loss (%)** | **TGI (%)** | **Max. BW loss (%)** |
| **TH-302 50mpk** | **67.6*** | **7.1** | **81*** | **0.6** |
| **Sunitinib 40mpk** | **72.9*** | **0** | **83*** | **0.1** |
| **Sunitinib 80mpk** | **77.7*** | **0** | **96*** | **0.5** |
| **Sunitinib 40mpk + TH-302** | **86.7*^{,b}** | **1.5** | **99*^{,a, b}** | **0.6** |
| **Sunitinib 80mpk + TH-302** | **96.6*^{,b}** | **1.2** | **102*^{,a, b}** | **3.3** |

| | | | | |
|---|---|---|---|---|
| *, p<0.05 vs. Vehicle a, p<0.05 vs. same dose Sunitinib monotherapy b, p<0.05 vs. TH-302 monotherapy | | | | |

**Table 3: TH-302 enhanced sorafenib monotherapy in xenograft tumors**

| | **HCC: PLC/PRF/5 (Sorafenib 20mpk)** | | | **RCC: 786-O (Sorafenib 30mpk)** | | | **NSCLC: H460 (Sorafenib 20mpk)** | | | **Melanoma: A375 (Sorafenib 20mpk)** | | |
|---|---|---|---|---|---|---|---|---|---|---|---|---|
| | **TGI (%)** | | **Max. BW loss (%)** | **TGI (%)** | | **Max. BW loss (%)** | **TGI (%)** | | **Max. BW loss (%)** | **TGI (%)** | | **Max. BW loss (%)** |
| **Vehicle** | | | **5.9** | | | **0** | | | **0** | | | **0** |
| **TH-302 50mpk** | **15** | | **6.3** | **15** | | **0** | **78** | ***** | **4.8** | **46** | ***** | **1.8** |
| **Sorafenib** | **56** | ***** | **5.6** | **38** | ***** | **0.4** | **74** | ***** | **4.8** | **67** | ***** | **3.1** |
| **Sorafenib + TH-302** | **81** | ***^{,a,b}** | **16.6** | **63** | ***^{,b}** | **6.3** | **94** | ***^{,a,b}** | **10.4** | **90** | ***^{,b}** | **8** |

| | | | | | | | | | | | | |
|---|---|---|---|---|---|---|---|---|---|---|---|---|
| *, a, b are as defined above. | | | | | | | | | | | | |

**Table 4: TH-302 enhanced everolimus monotherapy in 786-O RCC**

| | **TGI (%)** | **Max. BW loss (%)** |
|---|---|---|
| **Vehicle TH-302 50mpk (Day 1)** | **15.7** | **0 0.2** |
| **TH-302 50mpk (Day 8)** | **14.2** | **0** |
| **Everolimus 5mpk** | **48.8*** | **0** |
| **Everolimus 5mpk + TH-302 (Day 1)** | **84.7*^{,a, b}** | **0** |
| **Everolimus 5mpk + TH-302 (Day 8)** | **83.7 *^{,a, b}** | **0** |

| | | |
|---|---|---|
| *, a, b are as defined above. | | |

### Example 3. Clinical Administration of TH-302 with Sunitinib for the Treatment of Advanced Renal Cell Carcinomas, Gastrointestinal Stromal Tumors and Pancreatic Neuroendocrine Tumors

Clinical investigations demonstrate the safety, tolerability and clinically relevant disease responses of TH-302 in combination with sunitinib administered to patients with renal cell carcinoma, gastrointestinal stromal tumors or pancreatic neuroendocrine tumors in accordance with the methods of the invention. Sunitinib is administered orally daily on Day 1 through Day 28 of a 42 day cycle. TH-302 is administered as a 30 to 60 minute intravenous infusion once a week on Day 8, Day 15 and Day 22 of the 42 day cycle. Patients who successfully completed a 6-week treatment cycle without evidence of significant treatment-related toxicity or progressive disease are continued on treatment and can receive treatment for up to six cycles. In other embodiments, additional cycles may be administered.

One patient with advanced renal cell carcinoma has been treated at a TH-302 dose of 240 mg/m² in combination with a daily sunitinib dose of 50 mg.

While certain embodiments have been illustrated and described in the foregoing examples, it will be understood that changes and modifications can be made in the foregoing processes in accordance with ordinary skill in the art without departing from the present invention in its broader aspects as defined in the following claims.

## Claims

1. A composition comprising an antiangiogenic agent and a hypoxia activated prodrug for use in a method of treating a cancer patient in need of such treatment, the method comprising administering to a patient in need thereof, a therapeutically effective amount of the antiangiogenic agent and a therapeutically effective amount of the hypoxia activated prodrug to the patient, wherein the hypoxia activated drug is TH-302, and wherein the therapeutically effective amount of TH-302 is a once weekly dose of 480 mg/m² to 670 mg/m², wherein the antiangiogenic agent is selected from the group consisting everolimus, sunitinib, sorafenib, and pazopanib.

2. The composition for the use of claim 1, wherein administration of the hypoxia activated prodrug occurs only after administration of the antiangiogenic agent has resulted in an increased hypoxic fraction of the cancer.

3. The composition for the use of claim 2, wherein the first administration of the hypoxia activated prodrug is at least 7 days after the first administration of the antiangiogenic agent.

4. The composition for the use of any of claims 1 to 3, wherein the antiangiogenic agent and the hypoxia activated prodrug are administered sequentially.

5. The composition for the use of any of claims 1 to 4, wherein the cancer is selected from the group consisting of breast cancer, colorectal cancer, glioblastoma, astrocytoma, renal cell carcinoma, pancreatic cancer, sarcoma, hepatic cell carcinoma, gastrointestinal stromal tumor, and pancreatic neuroendocrine tumor.

6. The composition for the use of claim 5, wherein the antiangiogenic agent is pazopanib, wherein the cancer is selected from the group consisting of pancreatic cancer, renal cell carcinoma, and sarcoma.

7. The composition for the use of claim 5, wherein the antiangiogenic agent is sorafenib wherein the cancer is selected from the group consisting of hepatic cell carcinoma and renal cell carcinoma.

8. The composition for the use of claim 5, wherein the antiangiogenic agent is sunitinib, wherein the cancer is selected from the group consisting of gastrointestinal stromal tumor, renal cell carcinoma, and pancreatic neuroendocrine tumor.

9. The composition for the use of claim 2, wherein the hypoxic fraction of the cancer is measured prior to or after first administration of the antiangiogenic agent.

10. The composition for the use of claim 1, wherein the therapeutically effective amount of TH-302 is a once weekly dose of 575 mg/m².

## Patentansprüche

1. Zusammensetzung, die ein antiangiogenes Mittel und ein durch Hypoxie aktiviertes Prodrug zur Verwendung in einem Verfahren zum Behandeln eines Krebspatienten, der eine solche Behandlung benötigt, wobei das Verfahren ein Verabreichen an einen Patienten, der dies benötigt, einer therapeutisch wirksamen Menge des antiangiogenen Mittels und einer therapeutisch wirksamen Menge des durch Hypoxie aktivierten Prodrugs an den Patienten umfasst, wobei das durch Hypoxie aktivierte Arzneimittel TH-302 ist, und wobei die therapeutisch wirksame Menge von TH-302 eine einmal wöchentliche Dosis von 480 mg/m² bis 670 mg/m² ist, wobei das antiangiogene Mittel aus der Gruppe ausgewählt ist, die aus Everolimus, Sunitinib, Sorafenib und Pazopanib besteht.

2. Zusammensetzung für die Verwendung nach Anspruch 1, wobei die Verabreichung des durch Hypoxie aktivierten Prodrugs erst erfolgt, nachdem die Verabreichung des antiangiogenen Mittels zu einer erhöhten hypoxischen Fraktion des Krebses geführt hat.

3. Zusammensetzung für die Verwendung nach Anspruch 2, wobei die erste Verabreichung des durch Hypoxie aktivierten Prodrugs wenigstens 7 Tage nach der ersten Verabreichung des antiangiogenen Mittels stattfindet.

4. Zusammensetzung für die Verwendung nach einem der Ansprüche 1 bis 3, wobei das antiangiogene Mittel und das durch Hypoxie aktivierte Prodrug nacheinander verabreicht werden.

5. Zusammensetzung für die Verwendung nach einem der Ansprüche 1 bis 4, wobei der Krebs aus der Gruppe ausgewählt ist, die aus Brustkrebs, Darmkrebs, Glioblastom, Astrozytom, Nierenzellkarzinom, Bauchspeicheldüsenkrebs Sarkom, Leberzellkarzinom, gastrointestinalem Stromatumor, und pankreatischem neuroendokrinen Tumor besteht.

6. Zusammensetzung für die Verwendung nach Anspruch 5, wobei das antiangiogene Mittel Pazopanib ist, wobei der Krebs aus der Gruppe ausgewählt ist, die aus Bauchspeicheldüsenkrebs, Nierenzellkarzinom und Sarkom besteht.

7. Zusammensetzung für die Verwendung nach Anspruch 5, wobei das antiangiogene Mittel Sorafenib ist, wobei der Krebs aus der Gruppe ausgewählt ist, die aus Leberzellkarzinom und Nierenzellkarzinom besteht.

8. Zusammensetzung für die Verwendung nach Anspruch 5, wobei das antiangiogene Mittel Sunitinib ist, wobei der Krebs aus der Gruppe ausgewählt ist, die aus gastrointestinalem Stromatumor, Nierenzellkarzinom und pankreatischem neuroendokrinen Tumor besteht.

9. Zusammensetzung für die Verwendung nach Anspruch 2, wobei die hypoxische Fraktion des Krebses vor oder nach der ersten Verabreichung des antiangiogenen Mittels gemessen wird.

10. Zusammensetzung für die Verwendung nach Anspruch 1, wobei die therapeutisch wirksame Menge von TH-302 eine einmal wöchentliche Dosis von 575 mg/m² ist.

## Revendications

1. Composition comprenant un agent antiangiogénique et un précurseur de substance active activé par l'hypoxie à utiliser dans un procédé de traitement d'un patient cancéreux nécessitant un tel traitement, le procédé comprenant l'administration, à un patient le nécessitant, d'une quantité thérapeutiquement efficace de l'agent antiangiogénique et d'une quantité thérapeutiquement efficace du précurseur de substance active activé par l'hypoxie au patient, le médicament activé par l'hypoxie étant le TH-302, et la quantité thérapeutiquement efficace de TH-302 étant une dose hebdomadaire de 480 mg/m² à 670 mg/m², l'agent antiangiogénique étant choisi dans le groupe constitué par l'évérolimus, le sunitinib, le sorafénib et le pazopanib.

2. Composition à utiliser selon la revendication 1, l'administration du précurseur de substance active activé par l'hypoxie se produisant uniquement après que l'administration de l'agent antiangiogénique a entraîné une augmentation de la fraction hypoxique du cancer.

3. Composition à utiliser selon la revendication 2, la première administration du précurseur de substance active activé par l'hypoxie ayant lieu au moins 7 jours après la première administration de l'agent antiangiogénique.

4. Composition à utiliser selon l'une quelconque des revendications 1 à 3, l'agent antiangiogénique et le précurseur de substance active activé par l'hypoxie étant administrés séquentiellement.

5. Composition à utiliser selon l'une quelconque des revendications 1 à 4, le cancer étant choisi dans le groupe constitué par le cancer du sein, le cancer colorectal, le glioblastome, l'astrocytome, le carcinome à cellules rénales, le cancer du pancréas, le sarcome, le carcinome hépatocellulaire, la tumeur stromale gastro-intestinale, et la tumeur neuroendocrine du pancréas.

6. Composition à utiliser selon la revendication 5, l'agent antiangiogénique étant le pazopanib, le cancer étant choisi dans le groupe constitué par le cancer du pancréas, le carcinome à cellules rénales et le sarcome.

7. Composition à utiliser selon la revendication 5, l'agent antiangiogénique étant le sorafénib, le cancer étant choisi dans le groupe constitué par le carcinome hépatocellulaire et le carcinome à cellules rénales.

8. Composition à utiliser selon la revendication 5, l'agent antiangiogénique étant le sunitinib, le cancer étant choisi dans le groupe constitué par la tumeur stromale gastro-intestinale, le carcinome à cellules rénales et la tumeur neuroendocrine du pancréas.

9. Composition à utiliser selon la revendication 2, la fraction hypoxique du cancer étant mesurée avant ou après la première administration de l'agent antiangiogénique.

10. Composition à utiliser selon la revendication 1, la quantité thérapeutiquement efficace de TH-302 étant une dose hebdomadaire de 575 mg/m².
